# EUROPEAN PATENT SPECIFICATION

(11) **EP 1 775 235 B1**
(45) Date of publication and mention of the grant of the patent: **11.03.2009**
(21) Application number: 06121046.4
(22) Date of filing: 21.09.2006
(51) Int. Cl.: B65D 83/76

(54) **Liquid container**
Flüssigkeitsbehälter
Conteneur de liquide

(30) Priority: 14.10.2005 JP 2005300608; 21.04.2006 JP 2006118528
(43) Date of publication of application: 18.04.2007
(73) Proprietor: Kotobuki & Co. Ltd., Saitama Kawagoe (JP); Cosmopak International Limited, Roadtown Tortola (VG)
(72) Inventor: Kageyama, Hidehei, Kawagoe Saitama (JP)
(74) Representative: Haley, Stephen

(56) References cited:
- EP-A- 1 317 891
- EP-A- 1 396 210
- EP-A- 1 559 576
- DE-A1- 3 826 691
- FR-A- 2 753 613
- US-A- 2 035 372
- US-A- 2 595 097
- US-A1- 2004 005 185
- US-B1- 6 688 796

## Description

The present invention relates to a liquid container, according to the preamble of claim 1, that holds a liquid such as that for cosmetic, writing, medical or oral case use and has a liquid supply member exchangeably mounted to a front end thereof.

Conventionally, liquid containers of the type such as one described in Japanese Patent Laid-Open No. 9-192581 are known. The applicator container described in the above patent document includes an applicator container body that holds a coating solution therein, and an applicator mounted to a front end of the applicator container body to apply the coating solution to an object to be applied. The applicator is a tubular resilient member with a front end and a rear end and both of the ends are open. The applicator container body includes an outer barrel, a liquid introducing tube which is pressed into an front end of the outer barrel, an inner barrel which holds a coating solution and is axially movably arranged in the outer barrel, a valve device to open a valve to push the coating solution in the inner barrel into the liquid introducing tube when the inner barrel is pushed, a front barrel which is fixedly mounted to the liquid introducing tube, and a pipe which is attached by insertion to the front barrel, and the applicator is configured to be removably fitted in the pipe.

Pushing the inner barrel causes the valve to be opened to expel the coating solution into the liquid introducing tube, so that the coating solution can be distributed from the applicator to outside through the pipe. Since the applicator of the resilient member is removably fitted in the pipe, a user can constantly use a new clean applicator by removing a used applicator from the pipe and exchanging it into a new one at a proper time.

However, in the above conventional liquid container, when the inner barrel is pushed to open the valve, the coating solution in the inner barrel is expelled into the applicator, and when the internal barrel is released from rest at the pushed point, the coating solution which was not applied to the object to be applied is inevitably pulled back into the inner barrel. This means the coating solution which contacted an object or air and a previous applicator remains in the container even if the applicator is exchanged into a new one.

Thus, the conventional liquid containers have a problem that although the applicators can be exchanged, for example in order to use the applicators separately for each object to be applied with the liquid, since the liquids remain in the containers and mix with other liquids, users are not allowed to use containers completely separately for each object to be applied.

In viewing the foregoing, one object of the present invention is to provide a liquid contain which includes a liquid supply member that is exchangeably mounted thereto and prevents liquids in liquid supply members from being mixed each other after exchanging the liquid supply members.

US20040005185A discloses a cartridge-type applicator in which rotation of an actuator causes axial movement of a piston mechanism within a cartridge tank. US2595097A discloses a reservoir or fountain pen-type writing instrument in which rotation of an end cap causes axial movement of a piston within an ink containing cylinder. US2035372A discloses a fountain pen including a reciprocating plunger used to create a vacuum in the ink chamber so as to enable refilling the pen by drawing ink up through the nib.

The invention consists in a liquid container, comprising:
a container body that includes a body case having a tank section to hold a liquid therein and a front end port;
a liquid supply member coupled to a front end of the container body;
a piston that is advanced through the tank section; and
a piston advancing mechanism that has an operation member and causes the piston to be advanced through the tank section in response to the operation of the operation member;
wherein the liquid supply member is removably coupled to the container body, and the piston advancing mechanism causes the piston to be advanced only in the forward direction;
characterised in that the container body further comprises:
a tip element integrally mounted on the outer circumference of the body case front end port, wherein the tip element has a front end port and an annular groove that is formed in the front end surface radially outward from the front end port and the annular groove includes an outer circumferential surface provided with a female threaded portion, with which a male threaded portion of the liquid supply member is removably engaged.

The piston advancing mechanism can further comprise a piston rod which has a front end coupled to the piston, and extends backward from the front end with a threaded portion, and a piston rod guide which is threadedly engaged with the threaded portion of the piston rod, and one of the piston rod and the piston rod guide is rotatable only in one direction.

The piston advancing mechanism can further comprise a rotating cam mechanism which converts a pushing operation of the operation member into rotation of a rotating cam in one direction, and the rotation of the rotating cam is transmitted to one of the piston rod and the piston rod guide.

The rotating cam mechanism can comprise the rotating cam which is provided on an outer circumferential surface of the piston rod guide in a relatively non-rotatable manner, the operation member to move the rotating cam axially, a tail cap to axially movably guide the operation member, and to guide the rotating cam axially movably and rotatably only in one direction, and a return spring to bias the rotating cam backward.

Rotation of the operation member can be transmitted to the piston rod guide, and the operation member is limited to rotate only in one direction.

The piston advancing mechanism can further comprise a ratchet cylinder that is fixed to the container body, and the operation member has a front end provided with serrations, and the ratchet cylinder has a rear end provided with ratchet teeth that mate with the serrations and are retractable and protrusive axially.

The operation member can be provided on a side portion of the body case so as to project and retract with respect to the body case.

The piston advancing mechanism can further comprise a rotary member which rotates in a predetermined direction by pushing of the operation member, and rotates in an opposite direction by releasing the pushing, and a conversion mechanism for converting the rotation of the rotary member in only one direction into a forward travelling motion of the piston in an axial direction inside the tank section.

According to the present invention, since the liquid supply member is removably coupled to the container body of the liquid container, the liquid supply member can be exchanged with a new one as needed. Because the piston advancing mechanism moves a piston only forward or without causing any back flow of liquid, no liquid is pulled back in the direction to the tank, and no liquid which contacted objects to be applied with the liquid or air remains inside the liquid container after the used liquid supply member is exchanged with a new one.

These and other objects, features, aspects, and advantages of the present invention will become apparent to those skilled in the art from the following detailed description, which, taken in conjunction with the annexed drawings, discloses preferred embodiments of the present invention.

Referring now to the attached drawings which form a part of this original disclosure;
Fig. 1 is a longitudinal cross-section view of a liquid container of a first embodiment according the present invention;
Fig. 2a is a plan view of the tail cap in Fig. 1;
Fig. 2b is a front view of the tail cap in Fig. 1;
FIG. 2C is a longitudinal cross-section view of the tail cap in FIG. 1;
FIG. 3A is a plan view of the pushing member in FIG. 1;
FIG. 3B is a longitudinal cross-section view of the pushing member in FIG. 1;
FIG. 4A is a plan view of the rotating cam in FIG. 1;
FIG. 4B is a longitudinal cross-section view of the rotating cam in FIG. 1;
FIG. 4C is a view on arrow as seen from the arrow 4C in FIG. 4B;
FIG. 5A is a developed view explaining the movement of a rotating cam mechanism;
FIG. 5B is a developed view explaining the movement of a rotating cam mechanism;
FIG. 5C is a developed view explaining the movement of a rotating cam mechanism;
FIG. 6 is a longitudinal cross-section view of the piston rod guide in FIG. 1;
FIG. 7A is a plan view of the stopper cylinder in FIG. 1;
FIG. 7B is a longitudinal cross-section view of the stopper cylinder in FIG. 1;
FIG. 8 is an end view as seen along the line 8-8 in FIG. 1;
] FIG. 9 is a longitudinal cross-section view of a liquid container of a second embodiment according the present invention;
FIG. 10 is an exploded perspective view of the main parts of FIG. 9;
FIG. 11 is an end view as seen along the line 11-11 in FIG. 9; and
FIG. 12 is an end view as seen along the line 12-12 in FIG. 9.
FIG. 13 is a longitudinal cross-section view of a liquid container of a third embodiment according the present invention;

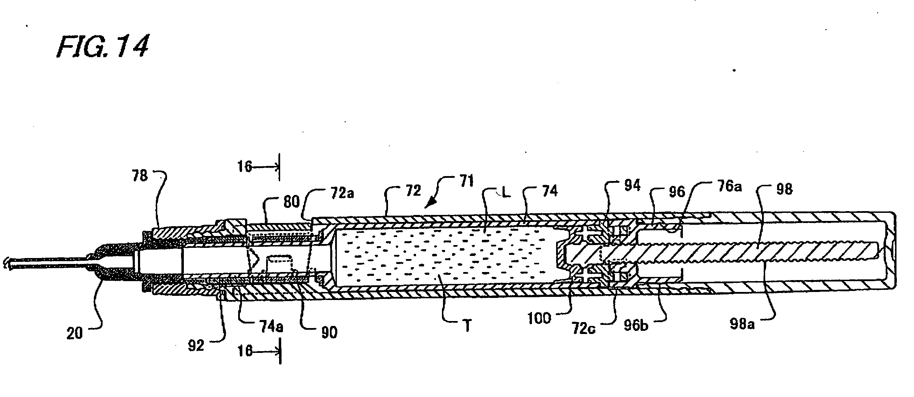

FIG. 14 is a longitudinal cross-sectional view showing a state in which a cap is removed and side knock manipulation is performed;
FIG. 15 is a cross-sectional view taken along the line 15-15 in FIG. 13;
FIG. 16 is a cross-sectional view taken along the line 16-16 in FIG. 14:
FIG. 17 is an exploded perspective view of major components of the advancing mechanism of Fig. 13;
FIG. 18A is a side view, FIG. 18B is a plan view, [0048]
FIG. 18C is a cross-sectional view taken along the line c-c in FIG. 18A, FIG. 18D is a view seen along the arrow d in FIG. 18A, and FIG. 18E is a view seen along the arrow e in FIG. 18A, of the rotary member;
FIGS. 19A is a plan view, and FIG. 19B is a sectional view taken along the line b-b in FIG. 19A, of an engaging member;
FIG. 20A is a plan view and FIG. 20B is a longitudinal cross-sectional view, of an inner cylinder;
FIG. 21A is a side view of a rotation stopping member and FIG. 21B is a view seen along the arrow b in FIG. 21A; and
FIG. 22A is a plan view, FIG. 22B is a longitudinal cross-sectional view of an inside screw member and FIG. 22C is a view seen along the arrow c in FIG. 22A.

Selected embodiments of the present invention will now be explained with reference to the drawings. It will be apparent to those skilled in the art from this disclosure that the following descriptions of the embodiments of the present invention are provided for illustration only and not for the purpose of limiting the invention as defined by the appended claims and their equivalents.

### (First Embodiment)

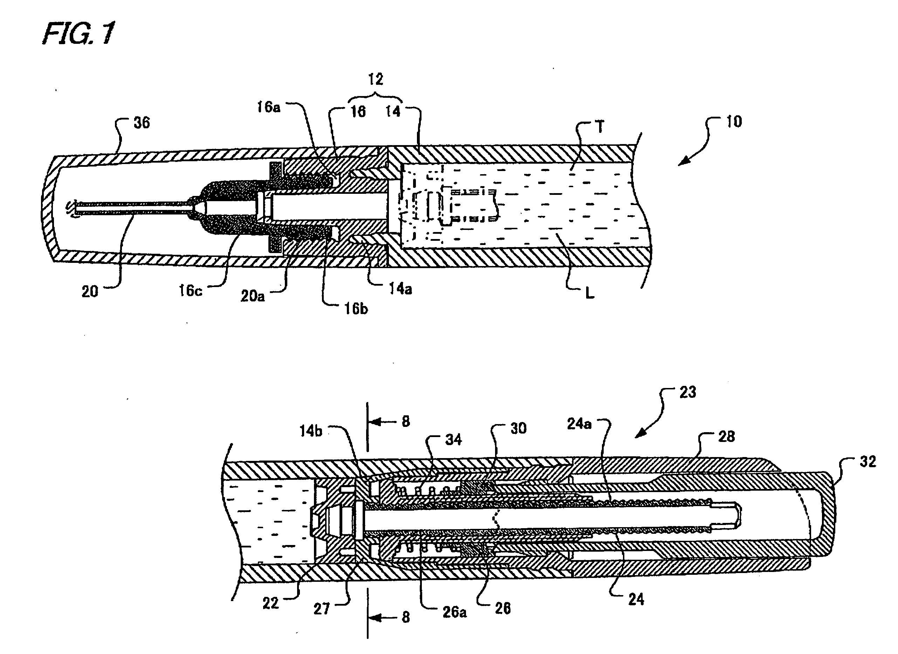

FIG. 1 is an entire view of a liquid container according to the present invention. In FIG. 1, a liquid container 10 generally includes a container body 12 having a tank section T that holds a liquid L for cosmetic, writing, medical use or oral case, an applicator 20 that is a liquid supply member mounted to the front of the container body 12, a piston 22 that is slidable through the tank section T, and a piston advancing mechanism 23 provided in the rear of the container body 12 to cause the piston 22 to be advanced forward.

The container body 12 includes a body case 14 which forms the tank section T and has a front end port 14a in a smaller diameter than that of the body case 14, and a tip element 16 that is integrally mounted on the outer circumference of the front end port 14a. The tip element 16 has a front end port 16c and an annular groove 16a that is formed in the front end surface radially outward from the front end port 16c. The annular groove 16a includes an outer circumferential surface provided with a female threaded portion 16b.

The applicator 20, which is a liquid supply member, has a rear end provided with a male threaded portion 20a to be threadedly engaged with the female threaded portion 16b of the tip element 16. The applicator 20 can be removed from the tip element 16 by disengaging the male threaded portion 20a from the female threaded portion 16b so that the applicator 20 is removable from the container body 12.

The liquid supply member may not onle be a syringe type applicator as shown in the figure, but also a brush, a bar of non-woveza material, or other member of any shape and configuration.

The liquid supply member can be removably mounted to the tip element 16 by a threaded engagement such as in this example, or by any other mounting structure including locking by fitting. The mounting structure of engagement or looking allows the liquid supply member to be easily removed from the tip element 16, and also prevents the liquid supply member from falling off from the tip element 16 while the liquid supply member is mounted to it. The applicator 20 can be removed from the front end of the tip element 16. which makes the removing easy with no liquid leaking.

When not in use, a cap 36 is removably attached to the tip element 16 to protect the applicator 20.

The piston advancing mechanism 23 comprises a piston rod 24 which has a front end coupled to the piston 22, and extends backward from the front end with a male threaded portion 24a along the circumferential surface thereof, and a piston rod guide 26 which has a front portion of the inner circumferential surface thereof in which a female threaded bore 26a is formed to be threadedly engaged with the male thread 24a of the piston rod 24, a stopper cylinder 27 through which the piston rod 24 passes in a non-rotatable manner relative to each other, a tail cap 28 coupled to the stopper cylinder 27, a rotating cam 30 mounted to the outer circumferential surface of the piston rod guide 26 in a non-rotatable manner relative to each other, a pushing member 32 projecting outward from the tail cap 28, and a return spring 34 to bias the rotating cam 30 backward. The pushing member 32 provides an operation member.

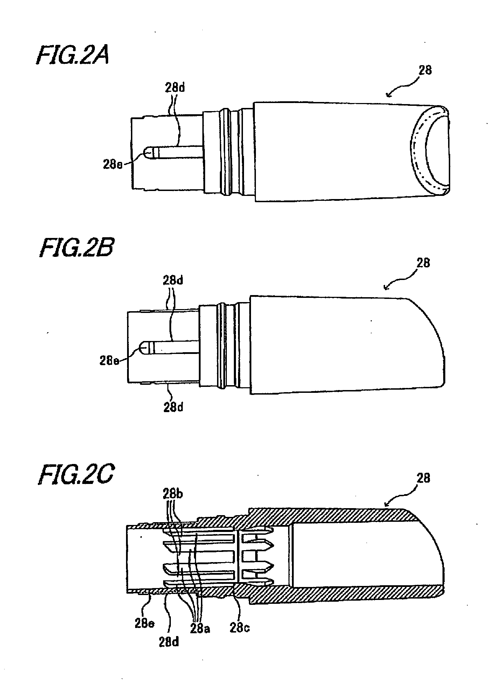

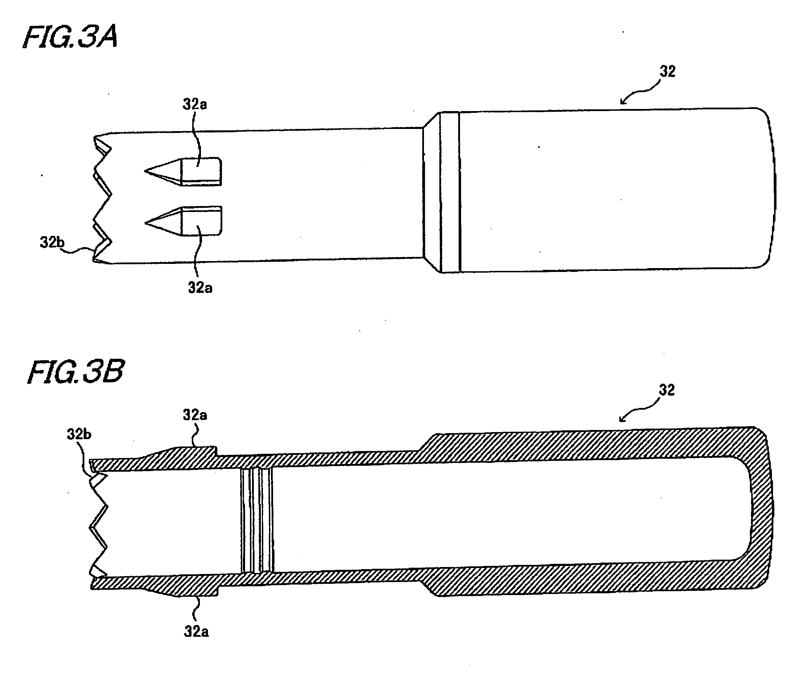

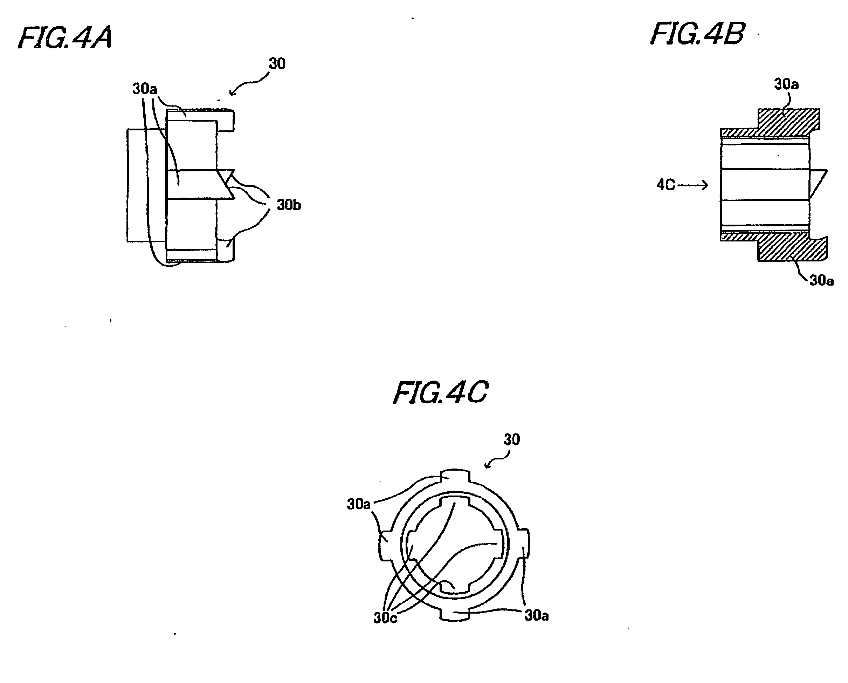

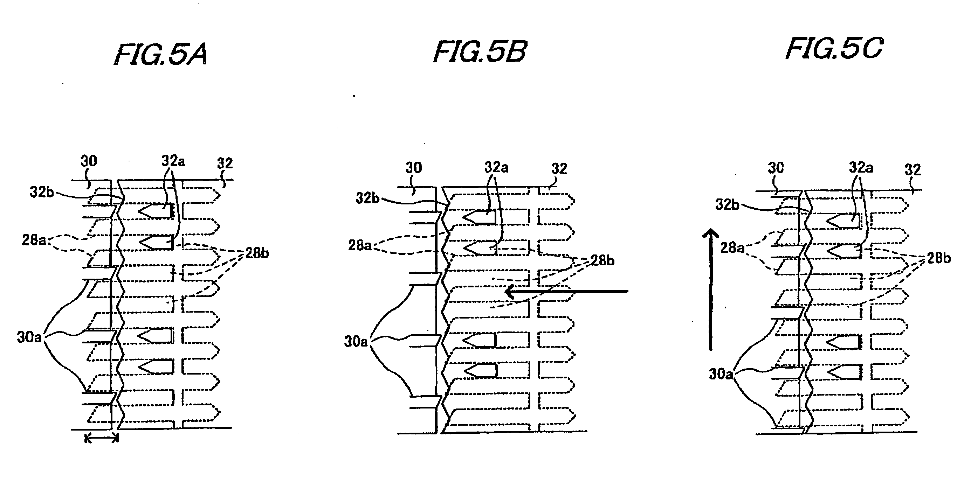

Now the configuration of the piston advancing mechanism 23 will be explained in detail. The tail cap 28, the pushing member 32, the rotating cam 30, and return spring 34 constitute a rotating cam mechanism, and as shown in FIG. 2C, a plurality of cam peaks 28a are provided circumferentially spaced from each other on the inner circumferential surface of the tail cap 28. Each of the cam peaks 28a has a front end with an axially sloped cam surface, and a rear end with an annular projection 28c to circumferentially connect the rear ends of the cam peaks 28a. Between the cam peaks 28a provided are cam grooves 28b to axially movably be fitted with projections 32a which are provided on the outer circumferential surface of the pushing member 32 (see FIG. 3). As shown in FIG. 3, the pushing member 32 has a front end with a serrated cam surface 32b. The rotating cam 30 has an outer circumferential surface provided with projections 30a (see FIG. 4) to be axially movably fitted with the cam grooves 28b of the tail cap 28. As shown in FIG. 4, each of the projections 30a of the rotating cam 30 has a rear end with an axially sloped cam surface 30b. When the pushing member 32 is pushed forward, the cam surface 32b forces the rotating cam 30 forward against the return spring 34, which in turn forces the projections 30a of the rotating cam 30 forward beyond the cam grooves 28b of the tail cap 28 and as the force of the pushing member 32 is decreased, the return spring 34 biases the cam surfaces 30b of the projections 30a on the rotating cam 30 to slide toward the cam surface 32b of the pushing member 32 and the cam grooves 28b adjacent to the cam surfaces of the cam peaks 28a on the tail cap 28, which results in the projections 30a being fitted in the cam grooves 28b (see FIG. 5). In this way, every time the pushing member 32 is pushed, the rotating cam 30 rotates by the amount to move to the adjacent cam grooves 28b.

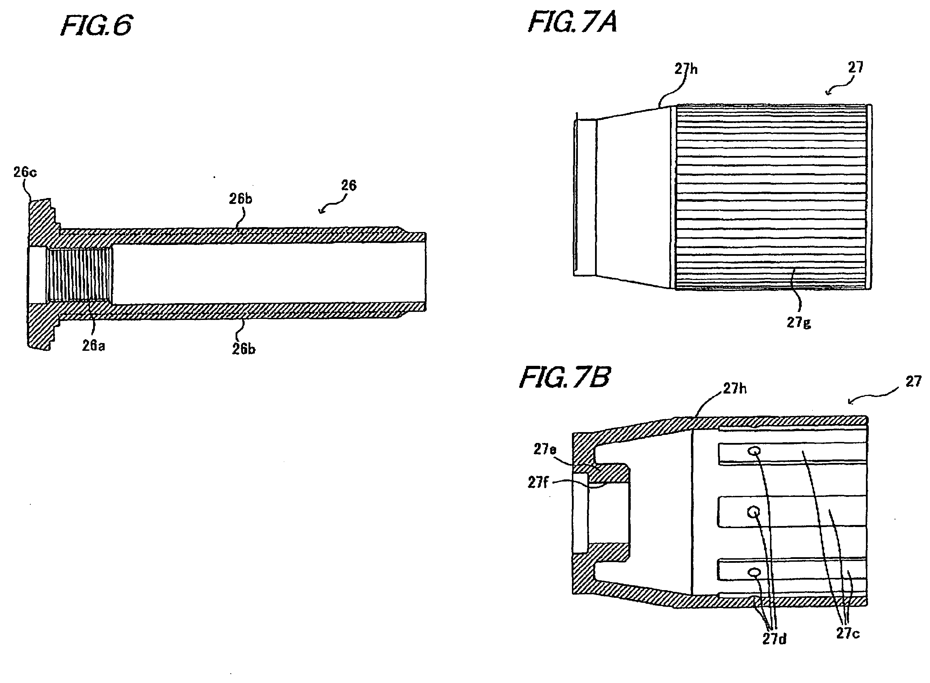

As shown in FIG. 4C, a plurality of longitudinal grooves 30c are formed in the inner circumferential surface of the rotating cam 30. Into the longitudinal grooves 30c, the longitudinal ribs 26b of the piston rod guide 26 (FIG. 6) are fitted to integrally rotate. As shown in FIG. 6, the front end of the piston rod guide 26 provides an annular projection 26c that project radially outward.

A plurality of longitudinal grooves 27c are formed in the rear portion of the inner circumferential surface of the stopper cylinder 27 as shown in FIG. 7. Into the longitudinal grooves 27c, the longitudinal rib 28d on the front portion of the outer circumferential surface of the tail cap 28 in FIG. 2 are inserted, and projections 27d which are formed on the longitudinal grooves 27c are inserted into the small holes 28e in the longitudinal rib 28d to integrally couple the stopper cylinder 27 and the tail cap 28.

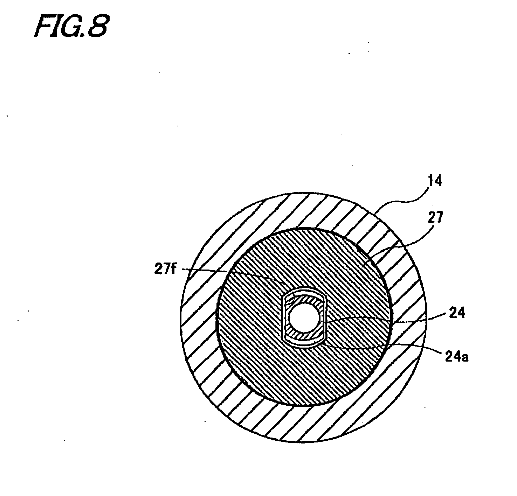

As shown in FIG. 8, the piston rod 24 has a non circular cross sectional shape, in this embodiment, an oval shape, and the stopper cylinder 27 has an stopper cylinder portion 27e with a through bore 27f formed therein with a cross sectional shape corresponding to that of the piston rod 24. The configuration that the piston rod 24 passes through the through bore 27f in the stopper cylinder portion 27e makes the piston rod 24 non-rotatable relative to the stopper cylinder 27. Moreover, the stopper cylinder 27 includes a knurled outer circumferential surface 27g that is fitted with the ribs formed on a part of the inner circumferential surface of the body case 14. The knurled structure provides a rotation stopper to make the stopper cylinder 27 non-rotatable relative to the body case 14. Of course, other than the knurled structure, structures such as fitting with ribs, fitting with polygons, fitting with keys and key grooves may be used as the rotation stopper between the stopper cylinder 27 and the body case 14. As described above, since the stopper cylinder 27 is non-rotatable relative to the piston rod 24, the piston rod 24 is non-rotatable relative to the body case 14. Also, the stopper cylinder 27 has a tapered surface 27h which is formed adjacent to the knurled outer circumferential surface 27g and the diameter of which is gradually reduced toward the front end thereof. The tapered surface 27h contacts a tapered surface 14b which is formed on the inner circumferential surface of the body case 14 and the diameter of which is gradually reduced toward the front end thereof. Moreover, the tail cap 28 is pressed in the body case 14 to be fixed relative to the body case 14, and the stopper cylinder 27 is held between the tapered surface 14b of the body case 14 and the tail cap 28 so that the stopper cylinder 27 is fixed relative to the body case 14. The annular projection 26c of the piston rod guide 26 is sandwiched between the stopper cylinder 27 and the tail cap 28 to be axially unmovable relative to the body case 14.

The piston advancing mechanism 23 can be assembled as an integral unit separately from the body case 14, and can be combined to the body case 14 by pressing the tail cap 28 of the unit into the rear end of the body case 14. After the combination of the piston advancing mechanism 23 and the body case 14, a liquid L is injected through the front end port of the body case 14, and the tip element 16 is pressed into the body case 14 to complete the assemble.

In use of the liquid container 10 of the above structure, the cap 36 is removed and the liquid is applied with the applicator 20, and in order to supply more liquid through the applicator 20, the pushing member 32 is pushed toward the container body 12. As described above, every time the pushing member 32 is pushed, the projections 30a of the rotating cam 30 rotates by the amount to move to the adjacent cam grooves 28b and the piston rod guide 26 integrally rotates. On the contrary, the piston rod 24 which is non-rotatable relative to the container body 12 does not rotate, which causes a relative rotation between the piston rod guide 26 and the piston rod 24, and the engagement between the female threaded bore 26a of the piston rod guide 26 and the male thread 24a of the piston rod 24 allows the piston rod 24 and the piston 22 to be advanced forward. As the piston 22 is advanced through the tank section T, the liquid L in the tank section T is forced toward the front end port 14a of the body case 14 to be supplied through the front end of the applicator 20 for use.

The liquid supply member, that is the applicator 20, may be needed to be exchanged. For example, if the liquid supply member is disposable, the liquid supply member is needed to be exchanged for each use. Or when the liquid supply member is worn off, it should be exchanged with a new one. Sometimes, depending on the purpose of the liquid supply, the liquid supply member is needed to be exchanged with another one with a different shape, a configuration, or a dimension.

The applicator 20 can be, if needed, exchanged with another one by disengaging the applicator 20 from the tip element 16. In this exchange, the liquid remained in the applicator 20 is also exchanged with the applicator 20, which improves the cleanliness and durability of the container. This is because, in this embodiment, due to the function of the piston advancing mechanism 23, the piston 22 is advanced forward, never backward, which prevents the liquid which once exited from the tank section T from flowing back into the tank section T.

In other words, in response to a pushing operation of the pushing member 32, the piston advancing mechanism 23 rotates the rotating cam 30 only in one direction, never in the opposite direction. This causes the piston rod 24 and the piston 22 coupled to it to be advanced only forward, because the piston rod 24 and the piston 22 are threadedly engaged to each other to be axially moved by the rotation of the rotating cam 30. Thus, the liquid will not return or flow back toward the tank section T by pulling, which prevents the liquid that once contacted objects to be applied with the liquid or air from remaining inside the container after the used applicator 20 is exchanged with another one.

Although in the above embodiment, the rotation of the rotating cam 30 in the rotating cam mechanism is transmitted to the piston rod guide 26 which is threadedly engaged with the piston rod 24, the rotation of the rotating cam 30 may be transmitted to the piston rod 24 to rotate the piston rod 24. In this case, the piston rod guide 26 may be fixedly provided on the tank section, so that the piston rod guide 26 is threadedly engaged with the piston rod 24 to operate in the same way.

### (Second Embodiment)

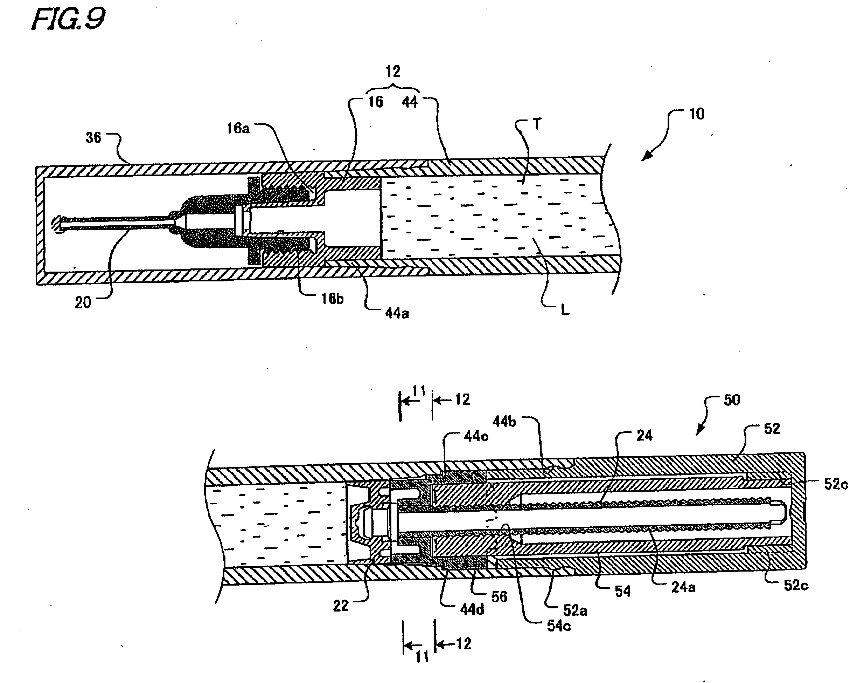

FIG. 9 is a view of a second embodiment of the present invention. In FIG. 9, the same numerals are used to designate the same or similar parts in the first embodiment, the description of which will be omitted.

A container body 12 of a liquid container 10 in this embodiment includes a body case 44 to form a tank section T having a front end port 44a the diameter of which is smaller than that of the body case 44, and a tip element 16 that is integrally mounted on the outer circumference of the front end port 44a.

A piston advancing mechanism 50 has a piston rod 24 which has a front end coupled to a piston 22 and extends backward from the front end with a male thread 24a along the circumferential surface thereof, an operation cylinder 52 which is mounted to the rear portion of the body case 44, a piston rod guide 54 formed with a female thread bore 54c to be threadedly engaged with the male thread 24a of the piston rod 24, and a ratchet cylinder 56 fixed in the rear portion inside of the body case 44 through which the piston rod 24 passes. The operation cylinder 52 provides an operation member. Preferably a slight play is provided axially between the piston rod 24 and the piston 22.

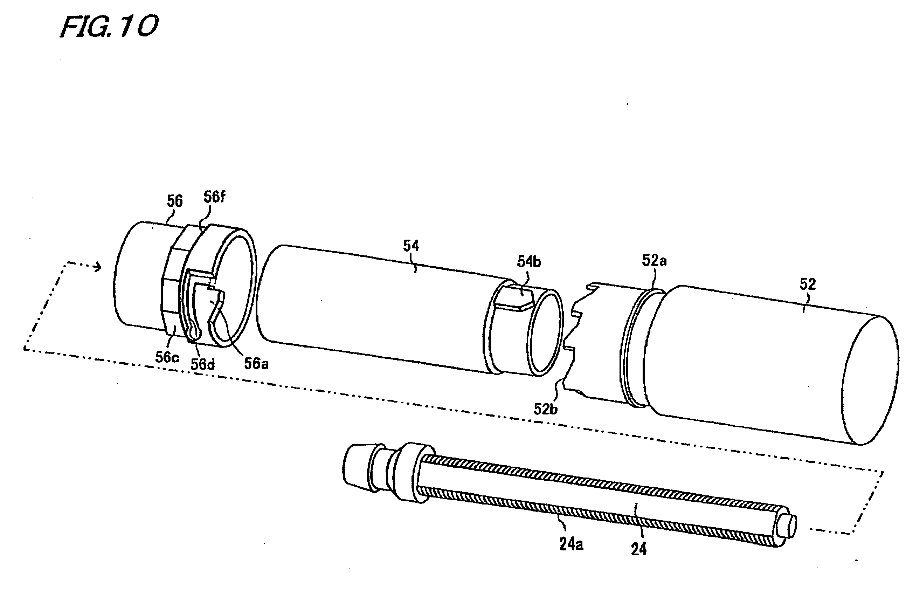

The operation cylinder 52 has an annular projection 52a which is formed on the front portion of the outer circumferential surface thereof. The annular projection 52a is fitted in an annular recess 44b which is formed in the rear portion of the inner circumferential surface of the body case 44 so that the operation cylinder 52 is mounted to the body case 44 rotatably relative to the body case 44. The operation cylinder 52 also has a plurality of longitudinal ribs 52c which are formed on the rear portion of the internal surface and project inward to provide a plurality of longitudinal grooves between the adjacent longitudinal ribs 52c. As shown in FIG. 10, the piston rod guide 54 has a longitudinal rib 54b at the rear end thereof, and the longitudinal rib 54b is fitted in the longitudinal grooves so that the operation cylinder 52 and the piston rod guide 54 integrally rotate.

Also as shown in FIG. 10, the operation cylinder 52 has serrations 52b at the front end thereof, while a ratchet cylinder 56 has a ratchet tooth 56a at the rear end thereof which are retractable and protrusive axially by elastically deforming with an L shaped slit 56d. The serrations 52b and the ratchet tooth 56a mate with each other.

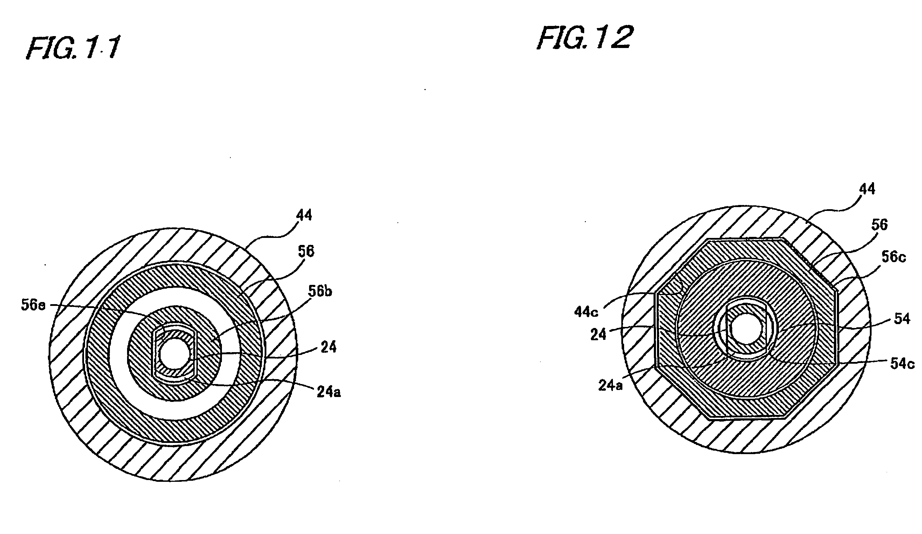

Also as shown in FIG. 10, the piston rod 24 has a non circular cross sectional shape with portions trimmed off from a circle, in this embodiment, an oval shape, and the ratchet cylinder 56 has an internal cylinder portion 56b with a through bore 56e formed therein with a cross sectional shape corresponding to that of the piston rod 24. As shown in FIG. 11, the configuration that the piston rod 24 passes through the through bore 56e in the internal cylinder portion 56b makes the piston rod 24 non-rotatable relative to the ratchet cylinder 56. Moreover, as shown in FIG. 12, the outer circumferential surface of the ratchet cylinder 56 includes a portion of a polygonal outer circumferential surface 56c, which is fitted in a polygonal bore 44c formed in an inner circumferential surface portion of the body case 44. The polygonal outer circumferential surface 56c and the polygonal bore 44c provide a rotation stopper structure to make the ratchet cylinder 56 non-rotatable relative to the body case 44, resulting in that the piston rod 24 is non-rotatable relative to the body case 44. Also, the ratchet cylinder 56 has an outer stepped portion 56f which is formed adjacent to the polygonal outer circumferential surface 56c to face forward, and the outer stepped portion 56f contacts an inner stepped portion 44d which is formed on the inner circumferential surface of the body case 44 to face backward. The ratchet cylinder 56 is held between the inner stepped portion 44d of the body case 44 and the serrations 52b of the operation cylinder 52, and is fixed relative to the body case 44.

In use of the liquid container 10 of the above structure, the cap 36 is removed and the liquid is applied with the applicator 20, and in order to supply more liquid through the applicator 20, the operation cylinder 52 is rotated relative to the body case 44.

Rotating the operation cylinder 52 relative to the body case 44 causes the piston rod guide 54 to rotate together with the operation cylinder 52. On the contrary, since the piston rod 24 is fitted in the through bore 56e of the ratchet cylinder 56 which is fixed to the body case 44, and does not rotate, it causes a relative rotation between the piston rod guide 54 and the piston rod 24, and the engagement between a female threaded bore 54c of the piston rod guide 54 and a male thread 24a of the piston rod 24 allows the piston rod 24 to be advanced forward. As the piston 22 is advanced through the tank section T, a liquid L in the tank section T is forced toward the front end port 44a of the body case 44 to be supplied through the front end of the applicator 20 for use.

On the rotation of the operation cylinder 52, the serrations 52b of the operation cylinder 52 slide on the sloped surface of the ratchet tooth 56a provided on the ratchet cylinder 56 to make the ratchet tooth 56a retract and protrude to achieve a rotation. This retraction and protrusion of the ratchet tooth 56a produces a click sound which gives a user moderation. If a user tries to turn the operation cylinder 52 in a wrong opposite direction, the serrations 52b of the operation cylinder 52 and the ratchet tooth 56a of the ratchet cylinder 56 mate with each other to prevent a relative rotation so that the user cannot rotate the operation cylinder 52. Accordingly, the operation cylinder 52 is consistently rotated only in a direction in which the piston 22 is advanced through the tank section T. The piston 22 is advanced through the body case 44 in response to the consumed and decreased amount of the liquid L. If the operation cylinder 52 is accidentally rotated a small amount, the operation cylinder 52 possibly returns to its original position because the serrations 52a cannot pass over the ratchet tooth 56a. However, even in such a case, only the piston rod 24 retracts relative to the piston 22 and the piston 22 does not retract, which prevents the back flow of the liquid.

As described above, also in this embodiment, since the operation cylinder 52 rotates only in one direction, and the piston 22 is advanced only forward, the liquid will not return or flow back toward the tank section T, which prevents the liquid that once contacted objects to be applied with the liquid or air from remaining inside the container after a used applicator 20 is exchanged with another one.

While only selected embodiments have been chosen to illustrate the present invention, it will be apparent to those skilled in the art from this disclosure that various changes and modifications can be made herein without departing from the scope of the invention as defined in the appended claims.

### (Third Embodiment)

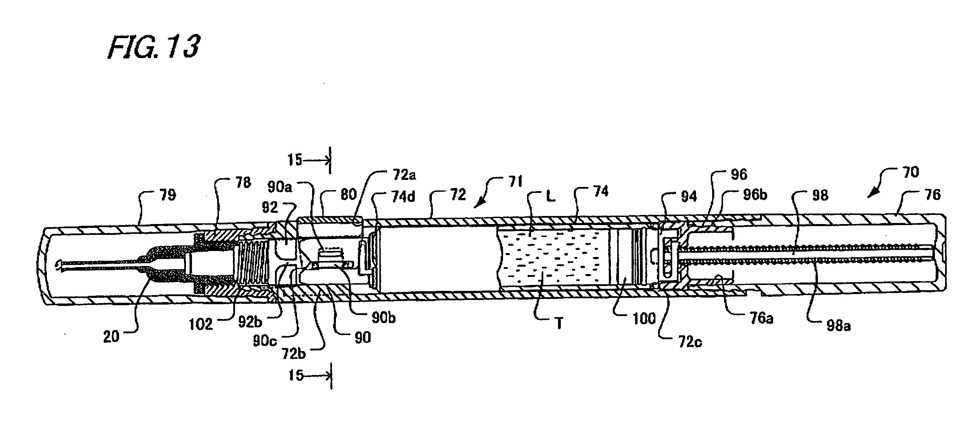

FIG. 13 is a view of a third embodiment of the present invention. In the drawing, the same numerals are used to designate the same or similar parts in the second embodiment, the description of which will be omitted.

A liquid container 70 includes a container body 71 which comprises body case 72, an inner barrel 74 provided concentrically inside the body case 72 and rotatably with respect to the body case 72, a rear barrel 76 mounted to a rear end of the body case 72, and a tip element 78 mounted to the tip end of the body case 72, and a cap 79 detachably fitted on the tip element78. An inside of the body case 72 and an inside of the inner cylinder 74 form a tank section T in which a liquid L for cosmetic, stationery, medical or oral care, etc, is housed. A cap 79 is detachably attached to the tip element 78 to protect the applicator 20 when it is not used.

An opening 72a is formed on a side surface of a tip end portion of the body case 72, a pushing member 80 for the user to manipulate is provided in the opening 72a, and the pushing member 80 is capable of projecting and retracting in an inward and outward direction of the body case 72,

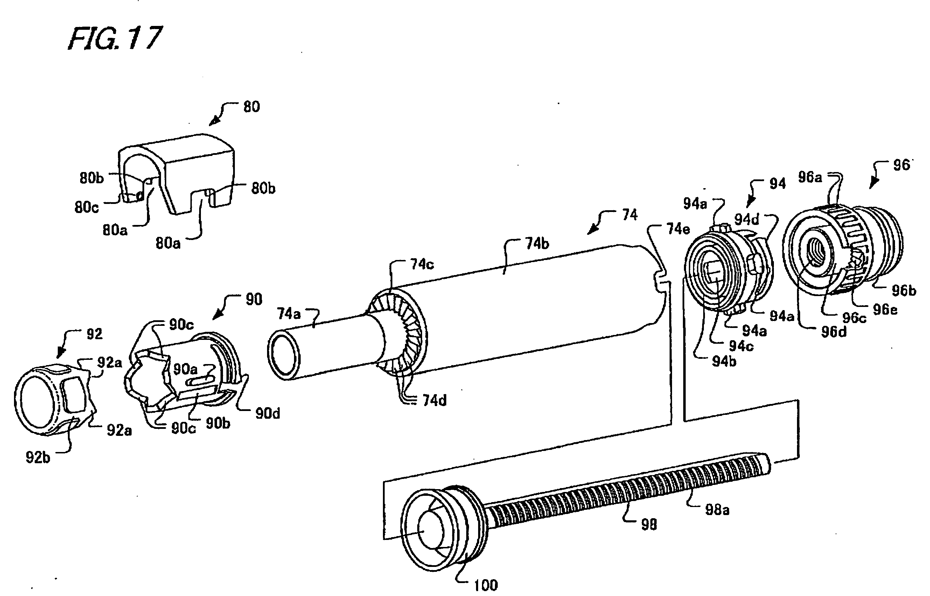

A piston 100 is slidably disposed inside the tank section T, and a piston advancing mechanism for advancing the piston 100 is provided. The piton advancing mechanism has, as shown in FIG. 17, the pushing member 80 capable of projecting from and retracting into the body case 72 constructing the container body 71, a rotary member 90 on which the pushing member 80 directly works, an engaging member 92 placed on a front side of the rotary member90, the inner cylinder 74, a rotation stopping member 94 provided at a rear end of the inner cylinder 74, an inside screw member 96 fixed to the body case 72, a piston rod 98 screwed into the inside screw member 96, a piston 100 connected to a tip end of the piston rod 98 and slidable inside the tank section T, and a return spring 102 (Fig. 13) as a biasing member for giving an urging force to the engaging member 92.

The inner cylinder 74 and the rotation stopping member 96 construct a transmitting member to which the rotation from the rotary member 90 is transmitted. The inner cylinder 74, the rotation stopping member 94, the inside screw member 96 and the piston rod 98 construct a conversion mechanism for converting the rotation of the rotary member 90 into a forward traveling motion of the piston 100 in the axial direction inside the body, and the engaging member 92 and the return spring 102 construct a rotation control mechanism for controlling a rotating direction of the rotary member 90. However, this example is one example, and it is possible to construct the transmission member, the conversion mechanism and the rotation control mechanism by optional members.

The respective members will be explained in detail hereinafter.

First, the pushing member 80 is in an approximately reversed U shape in a cross section, and rectangular notched portions 80a(Fig. 17) are formed at lower ends of its both side surfaces, and upper surfaces of the rectangular notched portions 80a become working surfaces 80b working on the rotary member 90. Slipping off preventing ribs 80c are formed on an inner surface of the pushing member 80, which are adjacent to the notched portions 80a.

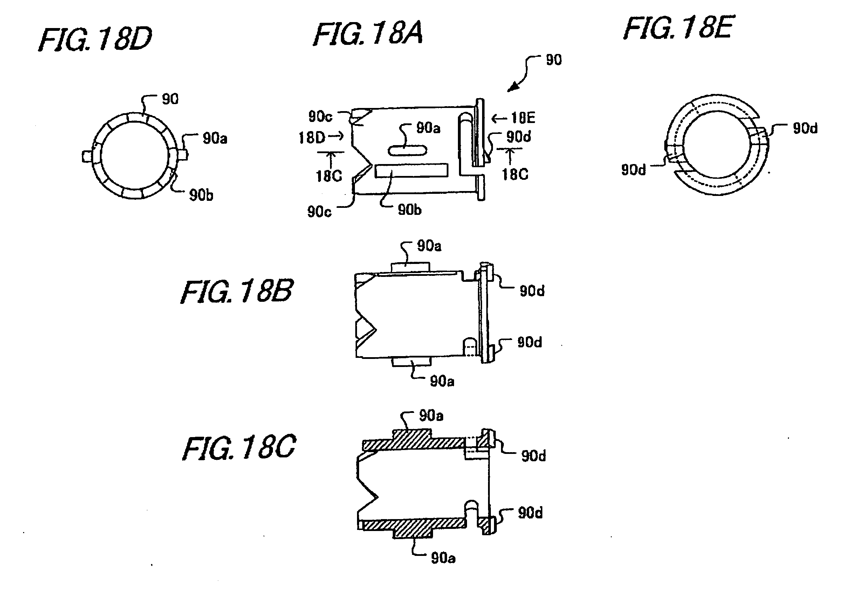

As shown in FIGS. 18A to 18E, the rotary member 90 is formed in a cylindrical shape, and the reduced diameter portion 74a (Fig. 17) of the inner cylinder 74 penetrates through an inside of the rotary member 90. Projections 90a and 90a are formed on the side surfaces of the rotary member 90 so that the working surfaces 80b of the pushing member 80 can press the projection 90a. Two projections 90a are formed to keep balance, but only one of the projections 90a always servos as a knock receiving projection for actually receiving the knock force of the pushing member 80.

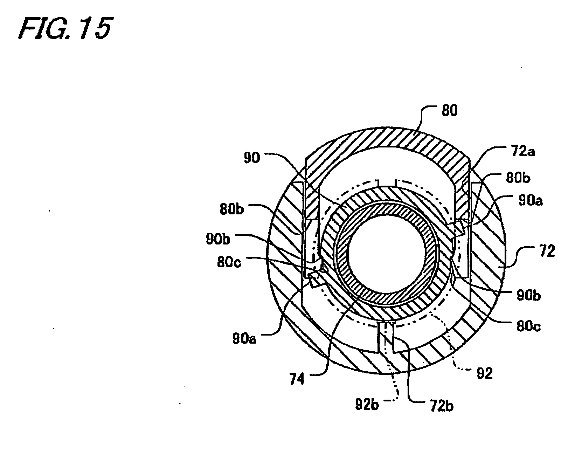

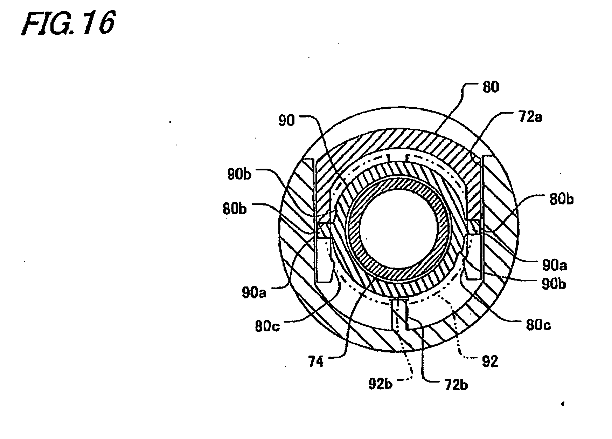

Slipping off preventing recessed portions 90b and 90b are formed in close vicinity of the projections 90a and 90a. The slipping off preventing recessed portion 90b engages with the slipping off preventing rib 80c when the pushing member 80 is not knocked. As for engagement of the slipping off preventing recessed portion 90b and the slipping off preventing rib 80c, their sectional shapes are set so that the slipping off preventing rib 80c is prevented from moving in the direction approaching the opening 72a but is allowed to move in the opposite direction approaching the opening 72a by the slipping off preventing recessed portion 90b (see FIG. 15 and FIG. 16).

A plurality of cam inclined surfaces 90c are formed at the tip end of the rotary member90, and the cam inclined surf aces 90c are inclined with respect to the axial direction. A plurality of ratchet teeth 90d elastically deformable in the axial direction are formed at the rear end of the rotary member 90.

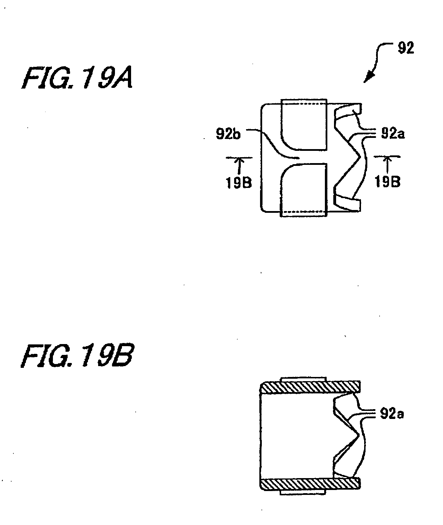

As shown in FIGS. 19A and 19B, an engaging member 92 disposed in front of the rotary member 90 is formed into a cylindrical shape, and the reduced diameter portion 74a of the inner cylinder 74 penetrates through the inside of the engaging member 92. Cam inclined surfaces 92a which can engage and slide in contact with a plurality of cam inclined surfaces 90c of the rotary member 90 are formed at the rear end of the engaging member 92. The cam inclined surfaces 92a are inclined with respect to the axial direction. A plurality of rotation stopping grooves 92b extending in the axial direction are formed on the peripheral surface of the engaging member 92, and a rotation stopping rib 72b formed on the inner peripheral surface of the body case 72 is fitted in the rotation stopping groove 92b, whereby the engaging member 92 can move in the axial direction with respect to the body case 72 but cannot rotate with respect to the body case 72.

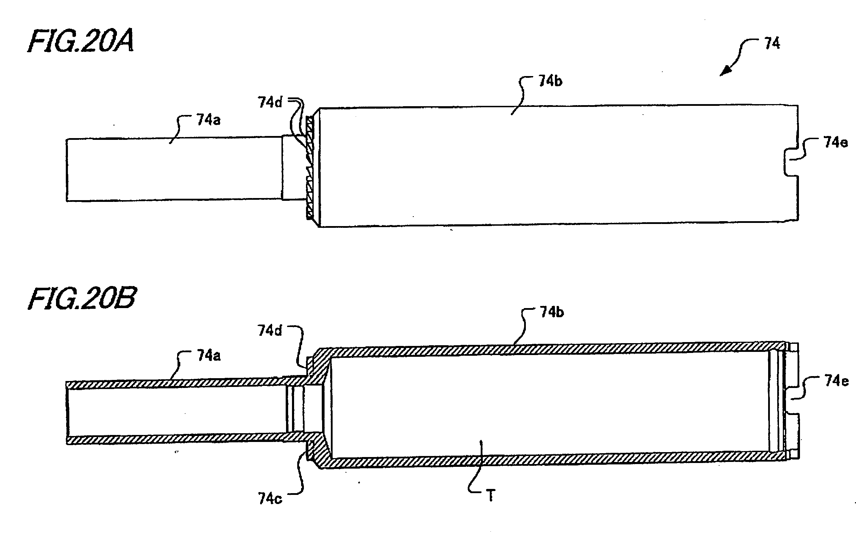

As shown in FIGS. 20A and 20B, the inner cylinder 74 which defines the tank section T and constructs the transmitting member has the reduced diameter portion 74a in the front end portion and an enlarged diameter portion 74b in the rear end portion. Sawteeth 74d are formed continuously in the circumferential direction on a boundary step portion 74c between the reduced diameter portion 74a and the enlarged diameter portion 74b. The saw teeth 74d can be meshed with the ratchet teeth 90d of the rotary member 90. The saw teeth 74d and the ratchet teeth 90d construct a first ratchet mechanism. A plurality of fitting notches 74e are formed at the rear end portion of the inner Cylinder 74.

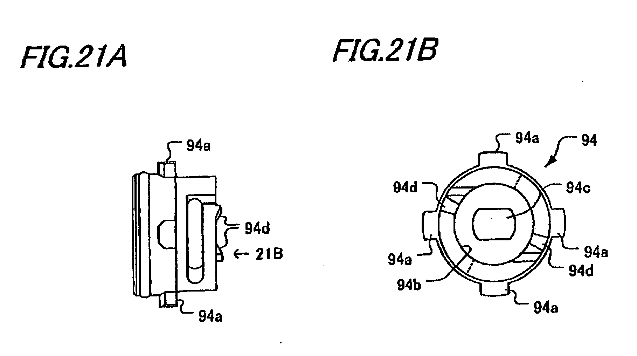

The rotation stopping member 94 provided at the roar end of the inner cylinder 74 forms a cylindrical shape. As shown in FIGS. 21A and 21B, a plurality of fitting protrusions 94a which fit into the fitting notches 74e of the inner cylinder 74 are formed on the outer peripheral surface of the rotation stopping member94. The fitting protrusions 94a fit into the fitting notches 74e, whereby the inner cylinder 74 and the rotation stopping member 94 are integrally connected. It is possible to construct the inner cylinder 74 and the rotation stopping member 94 as an integrated component, but it is desirable to construct them as separate components in molding.

An inner cylinder portion 94b is formed inside the rotation stopping member94, a non-circular hole 94c through which the piston rod 98 penetrates is formed in a center of the inner cylinder portion 94b. The cross-sectional shape of the piston rod 98 isformedintoanon-circular shape, andthenon-circular hole 94c corresponds to the cross-sectional shape of the piston rod 98, whereby the piston rod 98 is unrotatable with respect to the rotation stopping member 94.

A plurality of ratchet teeth 94d elastically deformable in the axial direction are formed at the rear end of the rotation stopping member 94.

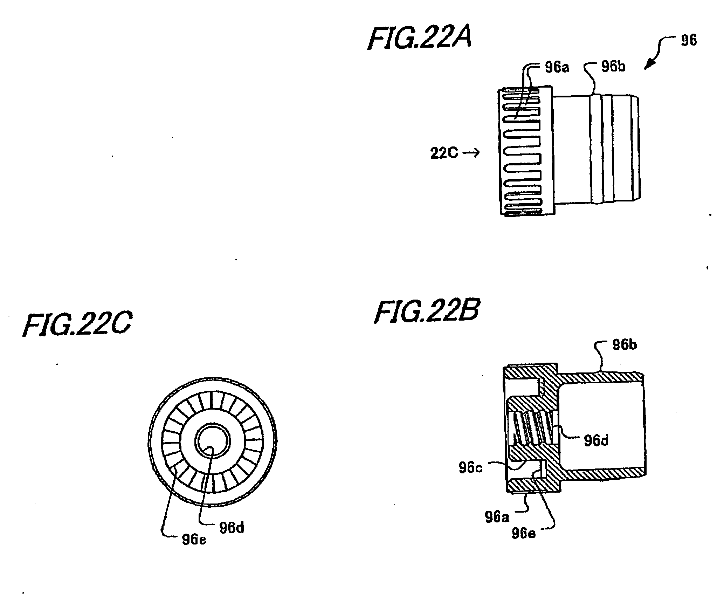

As shown in FIGS. 22A to 22C, the inside screw member 96 disposed behind the rotation stopping member 94 is formed into a cylindrical shape, and a number of rotation stopping ribs 96a extending in the axial direction are formed on an outer peripheral surface of the inside screw member 96. The rotation stopping rib 96a is fitted in the rotation stopping groove 72c formed on the body case 72, and thereby the inside screw member 96 is prevented from rotating with respect to the body case 72. Further, an annular fitting rib 96b is formed on the outer peripheral surface of the rear end portion of the inside screw member96. The fitting rib 96b is fitted into an annular fitting groove 76a formed in the rear barrel 76, and the inside screw member 96 is fixed to the body.

An inner cylinder portion 96c is formed inside the inside screw member96, and a female threaded hole 96d is formed in a center of the inner cylinder portion 96c. A male thread 98a formed on an outer peripheral surface of the piston rod 98 is screwed into the female thread hole 96d. The screw mechanism is constructed by the male thread 98a of this piston rod 98 and the female threaded hold 96d of the inside screw member 96.

Furthermore, sawteeth 96e are formed continuously in the circumferential direction on a surface extending in the circumferential direction between the outer cylinder portion and the inner cylinder portion 960 of the inside screw member 96, with facing to the front. A rear end portion of the rotation stopping member 94 is inserted between the outer cylinder portion and the inner cylinder portion 96c of the inside screw member96, and the sawteeth 96e can mesh with the ratchet teeth 94d of the rotation stopping member94. A second ratchet mechanism is constructed by the sawteeth 96e and the ratchet teeth 94d.

As shown in FIG. 15, in the normal state in which the pushing member 80 is not knocked, the cam inclined surface 90c of the rotary member 90 is engaged with the cam inclined surface 92a of the engaging member 92 so that the protrusions 90a and 90a are not horizontal but hold the inclined state. The engaging member 92 is biased toward the rotary member 90 by the return spring 102, and therefore the engagement of the cam inclined surface 90c and the cam inclined surface 92a is held. The knock receiving protrusion 90a which is one of the protrusions 90a is positioned in close vicinity to the working surface 80b of the pushing member 80.

As described the above, the transmitting member 74, 94 of the conversion mechanism is capable of connecting to and disconnecting from the rotary member 90, and is connected to the rotarymember 90 to rotate in the same direction with respect to rotation of the rotary member 90 in one direction, whereas disconnected from the rotary member 90 with respect to rotation in a direction opposite to the one direction so that the rotation is not transmitted to the transmitting member.

Furthermore, the first ratchet mechanism 74d, 90d of the conversion mechanism transmits the rotation in the one direction from the rotary member 90 to the transmitting member 74, 94, and does not transmit the rotation in the direction opposite to the one direction from the rotary member 90.

Furthermore, the second ratchet mechanism 96e, 94d of the conversion mechanism allows the rotation in the one direction of the transmitting member 74, 94 and inhibits the rotation in the direction opposite to the one direction.

Furthermore, the screw mechanism 98a, 96d of the conversion mechanism advance the piston rod 98 with respect to the rotation in a direction transmitted from the rotary member 90.

Furthermore, the cam mechanism 90c, 92a of the rotation control mechanism of the conversion mechanism moves the engaging member 92 in one direction in the axial direction by rotation of the rotary member 90 in the predetermined direction, and which moves the engaging member 92 in the opposite direction in the axial direction by rotation of the rotary member 90 in the opposite direction.

An operation of the liquid container 70 including the advancing mechanism constructed as above will be explained. First, when the liquid container 70 is used, the cap 79 is removed, and the liquid L is supplied by using the applicator 20. The liquid L from the tank section T is supplied to the applicator 20.

When the liquid L is fed from the tank section T, the pushing member 80 is pressed and pushed into the body case 72. The working surface 80b of the pushing member 80 presses the knock receiving protrusion 90a of the rotary member 90 downward in FIG. 15, and therefore the rotary member 90 is rotated in the clockwise direction in FIG. 15. When the rotary member 90 is rotated, the ratchet teeth 90d of the rotary member 90 are meshed with the sawteeth 74d of the inner cylinder 74, and this first ratchet mechanism transmits the clockwise rotation of the rotary member 90 to the inner cylinder 74. Therefore, the inner cylinder 74 rotates in the same direction and the rotation stopping member 94 is rotated together. Since the ratchet teeth 94d of the rotation stopping member 94 can slide on the sawteeth 96e of the inside screw member 96, and this second ratchet mechanism allows the rotation of the rotation stopping member 94, relative rotation movement occurs between the rotation stopping member 94 and the inside screw member 96. Since the piston rod 98 is unrotatable with respect to the rotation stopping member 94, the piston rod 98 rotates together with the rotation stopping member 94, and since the piston rod 98 is screwed into the female threaded hole 96d of the inside screw member 96, the piston rod 98 moves in the axial direction. Thus, the piston 100 connected to the piston rod 98 is pressed forward, and therefore the piston 100 slides within the tank section T and can feed the liquid L inside the tank section T forward.

Since the rotary member 90 rotates from the state shown in FIG. 15 to the state shown in FIG. 16 per one knock of the pushing member 80, and the piston rod 98 in synchronism with this also rotates by the same angle, the piston 100 moves in the axial direction by (angle of rotation/360) x pitch.

When this rotary member 90 rotates into the state shown in FIG. 16, the engaging member 92 cannot be rotated. Therefore the cam inclined surfaces 92a of the engaging member 92 are in sliding contact with the cam inclined surfaces 90c of the rotary member 90, and the engaging member 92 moves forward against the biasing force of the return spring 102.

When the knocking force to the pushing member 80 is released, the engaging member 92 is returned rearward by the restoring force of the return spring 102, the cam inclined surfaces 90c of the rotary member 90 slide in contact with the cam surfaces 92a of the engaging member 92, rotate in the counterclockwise direction in FIG. 16, and returns to the original position shown in FIG. 15.

At this time, since the ratchet teeth 94d of the rotation stopping member 94 are meshed with the sawteeth 96e of the inside screw member 96, and this second ratchet mechanism inhibits the counterclockwise rotation of the inner cylinder 74 and the rotation stopping member 94, the inner cylinder 74 and the rotation stopping member 94 cannot rotated with the rotary member 90. As a result, the ratchet teeth 90 of the rotary member 90 slides on the sawteeth 74d of the inner cylinder 74, only the rotary member 90 rotates and returns into the original state, and thereby the pushing member 80 returns to the upper position shown in FIG. 15.

Since in the state in which the pushing member 80 is not knocked, the engaging member 92 is biased rearward by the return spring 102, and the rotary member 90 is fixed in the posture shown in FIG. 15, the rotary member 90 does not rotate unexpectedly.

In this manner, every time the pushing member 80 is knocked, the piston 100 and the piston rod 98 move forward, and the liquid L which is the object to be fed can be fed from the applicator 20 placed at the tip end of the body. Since the liquid L can be fed by the side knock onto the pushing member 80, it is not necessary to change the grasp of the front barrel 12, it is not necessary to use both hands, and the manipulation can be simplified.

It is possible to construct the component constructed by a plurality of members in the above embodiment by a single member, or it is possible to construct the component constructed by a single member by a plurality of members.

The piston advancing mechanism rotates the inner cylinder 74 and the rotation stopping member 94 only in one direction, never in the opposite direction. This causes the piston rod 98 and piston 100 coupled to it to be advanced only forward. Thus, the liquid will not return or flow back toward the tank section T by pulling, which prevents the liquid that once contacted objects to be applied with the liquid or air from remaining inside the container after the used applicator 20 is exchanged with another one.

## Claims

1. A liquid container (10), comprising:
a container body (12) that includes a body case (14; 44) having a tank section (T) to hold a liquid (L) therein and a front end port (14a);
a liquid supply member (20) coupled to a front end of the container body;
a piston (22) that is advanced through the tank section; and
a piston advancing mechanism (23; 50) that has an operation member (32; 52) and causes the piston to be advanced through the tank section in response to the operation of the operation member;
wherein the liquid supply member is removably coupled to the container body, and the piston advancing mechanism causes the piston to be advanced only in the forward direction;
**characterised in that** the container body further comprises:
a tip element (16) integrally mounted on the outer circumference of the body case front end port (14a),
wherein the tip element has a front end port (16c) and an annular groove (16a) that is formed in the front end surface radially outward from the front end port (16c) and the annular groove includes an outer circumferential surface provided with a female threaded portion (16b), with which a male threaded portion (20a) of the liquid supply member is removably engaged.

2. The liquid container according to claim 1, wherein the piston advancing mechanism (23,50) further comprises: a piston rod (24) which has a front end coupled to the piston (22), and extends backward from the front end with a threaded portion (24a); and a piston rod guide (26; 54) which is threadedly engaged with the threaded portion of the piston rod, and one of the piston rod and the piston rod guide is rotatable only in one direction.

3. The liquid container according to claim 2, wherein the piston advancing mechanism (23) further comprises: a rotating cam mechanism which converts a pushing operation of the operation member (32) into rotation of a rotating cam (30) in one direction and the rotation of the rotating cam is transmitted to one of the piston rod (24) and the piston rod guide (26).

4. The liquid container according to claim 3, wherein the rotating cam mechanism comprises; the rotating cam (30) which is provided on an outer circumferential surface of the piston rod guide (26) in a relatively non-rotatable manner; the operation member (32) to move the rotating cam axially; a tail cap (28) to axially movably guide the pushing member, and to guide the rotating cam axially movably and rotatably only in one direction; and a return spring (34) to bias the rotating cam backward.

5. The liquid container according to claim 1 or claim 2, wherein rotation of the operation member (52) is transmitted to the piston rod guide (26), and the operation member is limited to rotate only in one direction.

6. The liquid container according to claim 5, wherein the piston advancing mechanism (50) further comprises a ratchet cylinder (56) that is fixed to the container body (12), and the operation member (52) has a front end provided with serrations (52b), and the ratchet cylinder has a rear end provided with ratchet teeth (56a) that mate with the serrations and are retractable and protrusive axially.

7. The liquid container according to claim 1 or claim 2, wherein the operation member (80) is provided on a side portion of the body case (72) so as to project and retract with respect to the body case.

8. The liquid container according to claim 7, further comprising: a rotary member (90) which rotates in a predetermined direction by pushing of the operation member (80), and rotates in an opposite direction by releasing the pushing; and a conversion mechanism (96, 98) for converting the rotation of the rotary member in only one direction into a forward traveling motion of the piston (100) in an axial direction inside the tank section.

## Patentansprüche

1. Flüssigkeitsbehälter (10), enthaltend:
einen Behälterkörper (12), der ein Körpergehäuse (14; 44) enthält, das einen Tankabschnitt (T), um eine Flüssigkeit (L) darin aufzunehmen, und einen vorderen Endanschluss (14a) hat;
ein Flüssigkeits-Zuführelement (20), das mit einem vorderen Ende des Behälterkörpers verbunden ist;
einen Kolben (22) der durch den Tankabschnitt geschoben wird; und
einen Kolben-Schiebemechanismus (23; 50), der über ein Betätigungselement (32; 52) verfügt und bewirkt, dass der Kolben durch den Tankabschnitt in Erwiderung auf die Betätigung des Betätigungselementes geschoben wird;
wobei das Flüssigkeits-Zuführelement lösbar mit dem Behälterkörper verbunden ist und der Kolben-Schiebemechanismus bewirkt, dass der Kolben lediglich in der Vorwärtsrichtung geschoben wird;
**dadurch gekennzeichnet, dass** der Behälterkörper weiterhin enthält:
ein Spitzenelement (16), das integral am Außenumfang des vorderen Endanschlusses (14a) des Körpergehäuses angebracht ist,
wobei das Spitzenelement einen vorderen Endanschluss (16c) und eine ringförmige Rille (16a) aufweist, die in der vorderen Stirnfläche radial nach außen gerichtet vom vorderen Endanschluss (16c) ausgebildet ist und die ringförmige Rille eine Außenumfangsfläche aufweist, die mit einem Gewindelochabschnitt (16b) versehen ist, mit dem ein Gewindestiftabschnitt (20a) des Flüssigkeits-Zuführelements lösbar in Eingriff steht.

2. Flüssigkeitsbehälter nach Anspruch 1, bei dem der Kolben-Schiebemechanismus (23, 50) weiterhin enthält: eine Kolbenstange (24), deren vorderes Ende mit dem Kolben (22) verbunden ist und die sich von der Stirnfläche mit einem Gewindeabschnitt (24a) nach hinten erstreckt; und eine Kolbenstangenführung (26; 54), die mit dem Gewindeabschnitt der Kolbenstange verschraubt ist, wobei entweder die Kolbenstange oder die Kolbenstangenführung lediglich in eine Richtung drehbar ist.

3. Flüssigkeitsbehälter nach Anspruch 2, bei dem der Kolben-Schiebemechanismus (23) weiterhin enthält: einen Drehnockenmechanismus, der eine Schiebetätigkeit des Betätigungselementes (32) in eine Drehung einer Drehnocke (30) in eine Richtung umwandelt, wobei die Drehung der Drehnocke entweder auf die Kolbenstange (24) oder die Kolbenstangenführung (26) übertragen wird.

4. Flüssigkeitsbehälter nach Anspruch 3, bei dem der Drehnockenmechanismus enthält: die Drehnocke (30), die an der Außenumfangsfläche der Kolbenstangenführung (26) in relativ nicht drehbarer Art und Weise angebracht ist; das Betätigungselement (32), das die Drehnocke axial dreht; eine Endkappe (28), die das Druckelement axial beweglich führt und die Drehnocke nur in einer Richtung axial beweglich und drehbar führt; und eine Rückholfeder (34), die die Drehnocke rückwärts gerichtet vorspannt.

5. Flüssigkeitsbehälter nach Anspruch 1 oder Anspruch 2, bei dem die Drehung des Betätigungselementes (52) auf die Kolbenführungsstange (26) übertragen wird und das Betätigungselement derart eingeschränkt ist, dass es sich in nur einer Richtung drehen kann.

6. Flüssigkeitsbehälter nach Anspruch 5, bei dem der Kolben-Schiebemechanismus (50) weiterhin einen Sperrzahnzylinder (56) enthält, der am Behälterkörper (12) befestigt ist, und das Betätigungselement (52) über ein vorderes Ende verfügt, das mit Zähnen (52b) versehen ist, und der Sperrzahnzylinder ein hinteres Ende hat, das mit Sperrzähnen (56a) versehen ist, die mit den Zähnen zusammenpassen und zurückziehbar sind sowie axial hervorragen.

7. Flüssigkeitsbehälter nach Anspruch 1 oder Anspruch 2, bei dem das Betätigungselement (80) auf einem Seitenabschnitt des Körpergehäuses (72) derart vorgesehen ist, dass es hervorragt und im Bezug auf das Körpergehäuse zurückgezogen ist.

8. Flüssigkeitsbehälter nach Anspruch 7, weiterhin enthaltend: ein Drehelement (90), das sich in einer vorbestimmten Richtung durch Drücken des Betätigungselementes (80) dreht und sich durch lösen des Drucks in einer entgegengesetzten Richtung dreht; und einen Umwandlungsmechanismus (96, 98), der die Drehung des Drehelementes in lediglich einer Richtung in einer Vorwärtsbewegung des Kolbens (100) in einer axialen Richtung innerhalb des Tankabschnittes umwandelt.

## Revendications

1. Récipient de liquide (10), comprenant :
un corps de récipient (12) qui inclut un boîtier de corps (14; 44) ayant une section de réservoir (T) pour contenir un liquide (L) dedans et un orifice d'extrémité avant (14a) ;
un élément d'alimentation en liquide (20) couplé à une extrémité avant du corps de récipient ;
un piston (22) qui s'avance à travers la section de réservoir ; et
un mécanisme d'avance de piston (23; 50) qui a un élément d'actionnement (32; 52) et fait avancer le piston à travers la section de réservoir en réponse à l'actionnement de l'élément d'actionnement ;
dans lequel l'élément d'alimentation en liquide est couplé de manière amovible au corps de récipient, et le mécanisme d'avance de piston fait avancer le piston seulement dans la direction vers l'avant ;
**caractérisé en ce que** le corps de récipient comprend en outre
un élément de pointe (16) monté d'un seul bloc sur la circonférence extérieure de l'orifice d'extrémité avant (14a) du boîtier de corps,
dans lequel l'élément de pointe a un orifice d'extrémité avant (16c) et une rainure annulaire (16a) qui est formée dans la surface d'extrémité avant radialement vers l'extérieur depuis l'orifice d'extrémité avant (16c) et la rainure annulaire inclut une surface circonférentielle extérieure pourvue d'une partie filetée femelle (16b), avec laquelle une partie filetée mâle (20a) de l'élément d'alimentation en liquide vient en prise de manière amovible.

2. Récipient de liquide selon la revendication 1, dans lequel le mécanisme d'avance de piston (23; 50) comprend en outre ; une tige de piston (24) qui a une extrémité avant couplée au piston (22), et s'étend vers l'arrière depuis l'extrémité avant avec une partie filetée (24a) ; et un guide de tige de piston (26; 54) qui est en prise par filetage avec la partie filetée de la tige de piston, et l'un de la tige de piston et du guide de tige de piston peut tourner seulement dans une direction.

3. Récipient de liquide selon la revendication 2, dans lequel le mécanisme d'avance de piston (23) comprend en outre : un mécanisme de came tournante qui convertit une opération de poussée de l'élément d'actionnement (32) en rotation d'une came tournante (30) dans une direction et la rotation de la came tournante est transmise à l'un de la tige de piston (24) et du guide de tige de piston (26).

4. Récipient de liquide selon la revendication 3, dans lequel le mécanisme de came tournante comprend : la came tournante (30) qui est placée sur une surface circonférentielle extérieure du guide de tige de piston (26) de manière à sensiblement ne pas tourner ; l'élément d'actionnement (32) pour déplacer la came tournante axialement ; un bouchon d'extrémité (28) pour guider la came tournante axialement et en rotation mobilement seulement dans une direction ; et un ressort de rappel (34) pour solliciter la came tournante vers l'arrière.

5. Récipient de liquide selon la revendication 1 ou 2, dans lequel la rotation de l'élément d'actionnement (52) est transmise au guide de tige de piston (26), et l'élément d'actionnement est limité pour tourner dans une seule direction.

6. Récipient de liquide selon la revendication 5, dans lequel le mécanisme d'avance de piston (50) comprend en outre un cylindre de cliquet (56) qui est fixé sur le corps de récipient (12), et l'élément d'actionnement (52) a une extrémité avant pourvue de dentelures (52b), et le cylindre de cliquet a une extrémité arrière pourvue de dents de cliquet (56a) qui coopèrent avec les dentelures et peuvent faire saillie et se rétracter axialement.

7. Récipient de liquide selon la revendication 1 ou 2, dans lequel l'élément d'actionnement (80) est placé sur une partie latérale du boîtier de corps (72) de façon à faire saillie et se rétracter par rapport au boîtier de corps.

8. Récipient de liquide selon la revendication 7, comprenant en outre : un élément tournant (90) qui tourne dans une direction prédéterminée en poussant l'élément d'actionnement (80), et tourne dans une direction opposée en relâchant la poussée ; et un mécanisme de conversion (96, 98) pour convertir la rotation de l'élément tournant dans seulement une direction en un mouvement de déplacement vers l'avant du piston (100) dans une direction axiale à l'intérieur de la section de réservoir.
